# DEMANDE DE BREVET EUROPEEN

(11) **EP 1 642 619 A1**
(43) Date de publication de la demande: **05.04.2006**
(21) Numéro de dépôt: 05292052.7
(22) Date de dépôt: 03.10.2005
(51) Int. Cl.: A61Q 5/00, A61K 8/04

(54) **Composition cosmétique capillaire à base de particules creuses et de polymère fixant non siliconé**

(30) Priorité: 04.10.2004 FR 0410447
(71) Demandeur: L'OREAL, 75008 Paris (FR)
(72) Inventeur: Mathonneau, Estelle, 75010 Paris (FR); Rollat-Corvol, Isabelle, 75017 Paris (FR)
(74) Mandataire: Bourdeau, Françoise

(57) **Abrégé**

La présente invention est relative à de nouvelles compositions cosmétiques capillaires comprenant des particules creuses de matériau organosiliconé et au moins un polymère fixant non siliconé. Elle vise également un procédé cosmétique, en particulier un procédé de fixation et/ou de maintien de la coiffure mettant en oeuvre lesdites compositions, ainsi que l'utilisation de ces compositions pour la fabrication d'une formulation cosmétique, destinée notamment au maintien et/ou à la mise en forme de la coiffure.

## Description

La présente invention est relative à de nouvelles compositions cosmétiques capillaires comprenant des particules creuses de matériau organosiliconé et au moins un polymère fixant non siliconé. Elle vise également un procédé cosmétique, en particulier un procédé de fixation et/ou de maintien de la coiffure mettant en oeuvre lesdites compositions, ainsi que l'utilisation de ces compositions pour la fabrication d'une formulation cosmétique, destinée notamment au maintien et/ou à la mise en forme de la coiffure.

Les produits de coiffage permettant de fixer la coiffure sont généralement des gels, des solutions ou des mousses contenant un polymère fixant. L'inconvénient lié à ce type de produit réside dans le fait que la tenue de la coiffure est limitée dans le temps et que si on utilise des compositions cosmétiques ayant une proportion de polymère plus importante, on ne parvient pas toujours à améliorer la résistance de la coiffure. D'autre part, le toucher cosmétique de ce type de produit est particulièrement peu esthétique : toucher collant.

On connaît par ailleurs des traitements permanents des fibres kératiniques. Ces traitements utilisent un agent réducteur et un agent oxydant, et nécessitent une mise sous tension mécanique des cheveux à l'aide d'un matériel d'enroulage, afin de conférer une mise en forme durable de la chevelure. Bien qu'ils permettent effectivement d'augmenter le volume de la chevelure, ces procédés présentent l'inconvénient de modifier la forme de la coiffure et le niveau de frisure, et de dégrader le toucher de la fibre.

Toutefois, il existe un besoin de disposer de produits capillaires permettant de conférer aux cheveux du maintien et un aspect brillant durable, sans apport de caractère chargé trop marqué. Ces propriétés ne sont pas apportées par les compositions de l'état de la technique.

La présente invention se propose de fournir une solution à cette attente.

En particulier, la Demanderesse vient de découvrir de façon surprenante que des compositions comprenant des particules creuses de matériau organosiliconé et au moins un polymère fixant non siliconé conféraient aux cheveux une brillance intense et persistante, et que les cheveux étaient particulièrement lisses au toucher.

Elle a également découvert que ces compositions pouvaient être formulées en milieu alcoolique.

D'autres objets de l'invention apparaîtront à la lecture de la description et des exemples qui suivent.

La présente invention a donc pour objet une composition cosmétique capillaire, caractérisée en ce qu'elle est comprend dans un milieu cosmétiquement acceptable, des particules creuses de matériau organosiliconé et au moins un polymère fixant non siliconé.

Un autre objet de l'invention concerne un procédé cosmétique capillaire, en particulier un procédé de fixation et/ou de maintien de la coiffure mettant en oeuvre les compositions selon l'invention.

L'invention a également pour objet l'utilisation des compositions selon l'invention pour conférer de la brillance aux cheveux.

Au sens de la présente invention, on entend par polymère fixant tout polymère permettant de donner une forme à la chevelure ou de maintenir cette forme.

La composition selon l'invention comprend des particules creuses.

### PARTICULES CREUSES EN MATERIAU ORGANOSILICONE

Les particules creuses sont en particulier des sphères ou des portions de sphères creuses constituées d'un matériau organosiliconé.

Lesdites particules ont avantageusement un diamètre moyen allant de 0,05 µm à 10 µm.

Les portions de sphères creuses utilisées dans la composition selon l'invention peuvent avoir la forme de sphères creuses tronquées, et ayant une section transversale en forme de fer à cheval ou d'arceau. Il s'agit notamment de demi sphères creuses.

Le matériau organosiliconé est, de préférence, un polysiloxane réticulé de structure tridimensionnelle ; il comprend de préférence, voire est constitué de, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif, un groupe organique non réactif, et de préférence un groupe organique non réactif.

Le groupe organique non réactif peut être un groupe alkyle en C₁-C₄, notamment un groupe méthyle, éthyle, propyle, butyle, ou un groupe phényle, et de préférence un groupe méthyle.

Le groupe organique réactif peut être un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano. De préférence, le groupe organique réactif peut être un groupe époxy, un groupe (méth)acryloxy, un groupe alkényle, un groupe mercaptoalkyle, aminoalkyle. Le groupe organique réactif comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.

Comme groupe (méth)acryloxy, on peut citer un groupe 3-méthacryloxypropyl, un groupe 3-acryloxypropyl.

Comme groupe alkényle, on peut citer un groupe vinyl, allyl, isopropényl.

Comme groupe mercaptoalkyle, on peut citer un groupe mercaptopropyl, mercaptoéthyl.

Comme groupe aminoalkyle, on peut citer un groupe 3-(2-aminoéthyl)aminopropyl, un groupe 3-aminopropyl, un groupe N,N-diméthylaminopropyl.

Comme groupe halogénoalkyle, on peut citer un groupe 3-chloropropyl, un groupe trifluoropropyl.

Comme groupe glycéroxy, on peut citer un groupe 3-glycéroxypropyl, un groupe 2-glycéroxyéthyl.

Comme groupe uréido, on peut citer un groupe 2-uréidoéthyl.

Comme groupe cyano, on peut citer un groupe cyanopropyl, cyanoéthyl.

De préférence, dans le motif de formule (II), R¹ désigne un groupe méthyle.

Avantageusement, le matériau organosiliconé comprend les motifs (I) et (II) selon un rapport molaire motif (I) / motif (II) allant de 30/70 à 50/50, de préférence allant de 35/65 à 45/55.

Les particules de matériau organosiliconé sont notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁₋C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

Pour les groupements X et Y des composés (III) et (IV) :
Comme groupe alcoxy en C₁-C₄, on peut citer les groupes méthoxy, éthoxy ;
Comme groupe alkoxyéthoxy renfermant un groupe alcoxy en C₁-C₄, on peut citer les groupes méthoxyéthoxy, butoxyéthoxy ;
Comme groupe alcoxy en C₂-C₄, on peut citer les groupes acétoxy, propioxy ;
Comme groupe N,N-dilakylamino renfermant un groupement alkyle en C₁-C₄, on peut citer les groupes diméthylamino, diéthylamino ;
Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemple de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemple de composés de formule (IV) comportant un groupe R organique réactif, on peut citer :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréido comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques - tels que l'hydroxyde de sodium, l'hydroxyde de potassium, le carbonate de sodium, l'hydrogénocarbonate de sodium, ou des amines (telles qu'ammoniaque, triméthylamine, triéthylamine, hydroxyde de tétraméthylammonium) - ou des catalyseurs acides, choisis parmi les acides organiques - tels que l'acide citrique, l'acide acétique, l'acide méthanesulfonique, l'acide p-toulène sulfonique, l'acide dodécylbenzènesulfonique, l'acide dodécylsulfonique - ou minéraux - tels que l'acide chlorhydrique, l'acide sulfurique, l'acide phosphorique. Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

Les particules obtenues (ou les sphères) ont de préférence un diamètre moyen allant de 0,05 à 10 µm.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788.

Des portions de sphères creuses en forme de fer à cheval sont aussi décrites dans la demande JP-A-2000-191789.

La figure annexée illustre une particule en forme de bol en coupe transversale.

Avantageusement, comme il ressort de cette figure, ces portions creuses sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

Les dimensions mentionnées ci-dessus sont obtenues en calculant la moyenne des dimensions de cent particules choisies sur une image obtenue au microscope électronique à balayage.

Comme particules utilisables selon l'invention, on peut citer notamment :les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,2 µm et d'épaisseur 150 nm (particules vendues sous la dénomination NLK-506 par la société Takemoto Oil & Fat) ;
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,5 µm et d'épaisseur 350 nm ;
les particules constituées de l'organosilicone réticulé TAK-110 (polymère réticulé méthylsilanol/silicate) de la société TAKEMOTO OIL & FAT , en forme de bol , de largeur 0,7 µm, de hauteur 0,35 µm et d'épaisseur 100 nm ;
les particules constituées de l'organosilicone réticulé TAK-110 (polymère réticulé méthylsilanol/silicate) de la société TAKEMOTO OIL & FAT , en forme de bol , de largeur 7,5 µm, de hauteur 3,5 µm et d'épaisseur 200 nm.

Les particules, notamment les portions de sphères creuses, peuvent être présentes dans la composition selon l'invention, en une teneur allant de 0,01 % à 50 % en poids, par rapport au poids total de la composition, de préférence allant de 0,1 % à 30 % en poids, et préférentiellement allant de 1 % à 15 % en poids.

### POLYMERES FIXANTS NON SILICONES

Les polymères fixants non siliconés convenant dans l'invention sont notamment choisis parmi les polymères fixants non siliconés cationiques, anioniques, amphotères, non ioniques et leurs mélanges.

Les polymères fixants non siliconés cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 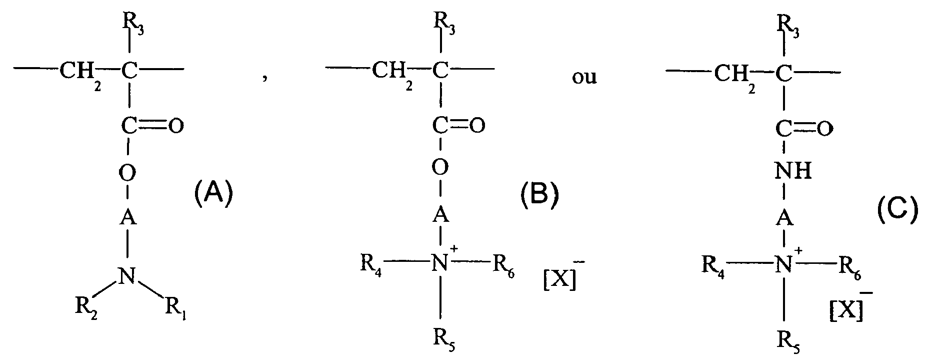 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français Nos 2 077 143 et 2 393 573,
   - les polymères à chaîne grasse et à motif vinylpyrrolidone, tels que les produits vendus sous la dénomination Stylène W20 et Stylène W10 par la société ISP,
   - les terpolymères méthacrylate de diméthylaminoéthyle/vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination "GAFQUAT® HS 100" par la société ISP ;
(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL ;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.
   Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants non siliconés anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 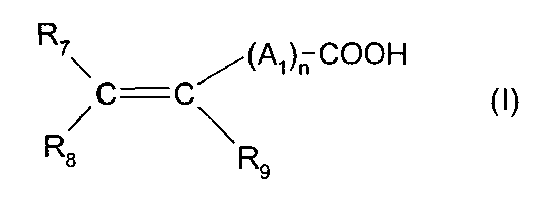 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants non siliconés anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁₋C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique a- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN
   ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, a-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse.
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

Comme autre polymère fixant non siliconé anionique utilisable selon l'invention, on peut citer le polymère anionique séquencé branché vendu sous la dénomination FIXATE G100 par la société NOVEON.

Selon l'invention, les polymères fixants non siliconés anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination FIXATE G100 par la société NOVEON.

Parmi les polymères fixants non siliconés anioniques cités ci-dessus, on préfère plus particulièrement utiliser dans le cadre de la présente invention les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination FIXATE G100 par la société NOVEON.

Les polymères fixants non siliconés amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-a, β-dicarboxylique dont l'un des groupements carboxyliques a été amené à réagir avec une polyamine comportant un ou plusieurs groupements amine primaire ou secondaire.

Les polymères fixants non siliconés amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle
   ou diéthyle.
   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/ butylaminoethyl methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale :

   ⁅ CO―R₁₀―CO―Z⁆ (II)

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 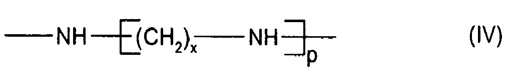 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 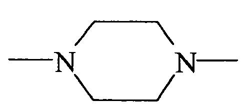
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 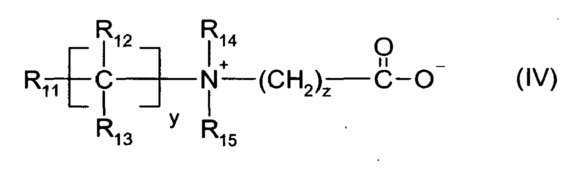 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 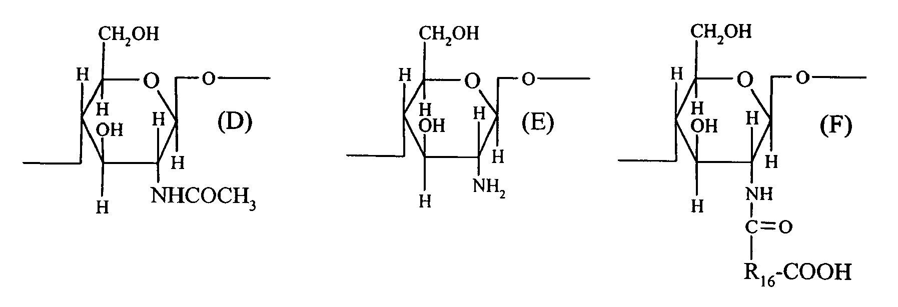 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 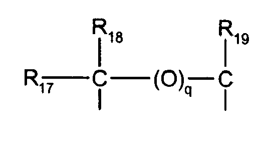 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 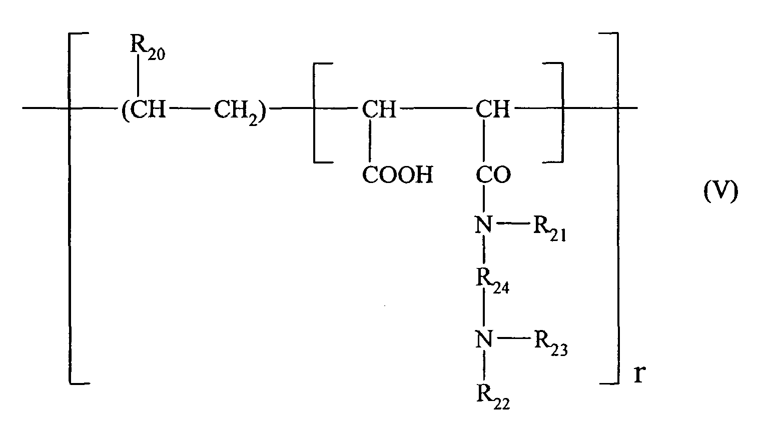 dans laquelle R₂₀ représente un atome d'hydrogène, un groupe CH₃0, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂₋CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 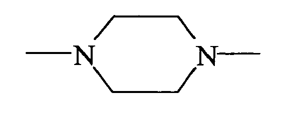 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 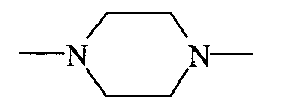 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants non siliconés amphotères décrits ci-dessus, les plus particulièrement préférés selon l'invention sont ceux de la famille (3) tels que les copolymères dont la dénomination CTFA est Octylacrylamide/ acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER® Z301 par la société SANDOZ.

Les polymères fixants non siliconés non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyle des polymères non ioniques mentionnés ci-dessus ont, de préférence, de 1 à 6 atomes de carbone.

On peut également utiliser comme polymères fixants non siliconés, des polyuréthanes fonctionnalisés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer le produit commercialisé sous la dénomination LUVISET PUR® par la société BASF.

Dans les compositions de l'invention, la teneur en polymère fixant non siliconé est généralement comprise entre 0,01 et 20%, de préférence entre 0,05 et 15%, et encore plus préférentiellement entre 0,1 et 10% en poids du poids total de la composition.

### COMPOSITION

Le milieu cosmétiquement acceptable est de préférence constitué par de l'eau ou par un ou plusieurs solvants cosmétiquement acceptable tels que des alcools ou par des mélanges eau-solvant(s). Ces solvants sont de préférence des alcools en C1-C4.

Parmi ces alcools, on peut citer l'éthanol, l'isopropanol, l'éthanol étant particulièrement préféré.

La composition selon l'invention peut contenir en outre au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques, amphotères ou zwitterioniques autres que les polymères fixants non siliconés décrits ci-dessus, les huiles minérales, végétales ou synthétiques, les agents et tout autre additif classiquement utilisé dans les compositions cosmétiques, tels que les agents antipelliculaires, les agents anti-chute, les colorants, les pigments, les agents hydratants tels que la glycérine et les autres polyols, les réducteurs, les oxydants.

Ces additifs sont présents dans la composition à des teneurs avantageusement comprises entre 0,001 et 30% en poids du poids total de la composition. La quantité précise de chaque additif est fonction de sa nature et est déterminée facilement par l'homme du métier, et dépendra de l'application capillaire choisie.

Bien entendu, l'homme du métier veillera à choisir le ou les additifs de manière que les propriétés avantageuses de la composition conforme à l'invention ne soient pas, ou substantiellement pas, altérées par l'addition envisagée.

Les compositions de l'invention peuvent être utilisées pour la fabrication de nombreux produits capillaires, comme par exemple, des produits pour la fixation et/ou le maintien des cheveux, des produits de conditionnement ou encore des produits de soin des cheveux.

Ces compositions peuvent être conditionnées sous diverses formes, notamment dans des flacons pompes ou dans des récipients aérosols, afin d'assurer une application de composition sous forme vaporisée ou sous forme de mousse. De telles formes de conditionnement sont indiquées, par exemple, lorsque l'on souhaite obtenir un spray, une laque, ou une mousse pour la fixation ou le traitement des cheveux. Les compositions conformes à l'invention peuvent également se présenter sous la forme de crèmes, de gels, d'émulsions, de lotions, ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyléther.

Avantageusement, l'agent propulseur est présent à une concentration comprise entre 5 et 90% en poids par rapport au poids total au poids total de la composition dans le dispositif aérosol, et plus particulièrement à une concentration comprise entre 10 et 60%.

Les exemples suivants sont destinés à illustrer l'invention.

### Exemple 1

| Formule de l'invention (formule 1) | |
|---|---|
| Hydroxypropyl guar | 1.0% |
| Demi sphère NLK-500 (TAKEMOTO) | 1.0% |
| Acrylate de butyle/acide acrylique/acide méthacrylique | 3.0% |
| Conservateur | 0.4% |
| Eau | 94.6% |

| Formule témoin 1 (formule 2) | |
|---|---|
| Hydroxypropyl guar | 1.0% |
| Acrylate de butyle/acide acrylique/acide méthacrylique | 3.0% |
| Conservateur | 0.4% |
| Eau | 96.1% |

On applique 1g de formule 1 sur 2.5 g de cheveux châtains européens.
On applique 1g de formule témoin (formule 2) sur 2.5 g de cheveux châtains européens de même nature.
La mèche avec la formule 1 est plus fixée.

### Exemple 2

| Formule de l'invention (formule 3) | |
|---|---|
| Gomme de Xanthane | 0.2% |
| Demi sphère NLK-500 (TAKEMOTO) | 1.0% |
| Acrylate de butyle/acide acrylique/acide méthacrylique | 3.0% |
| Conservateur | 0.4% |
| Eau | 95.4% |

| Formule témoin (formule 4) | |
|---|---|
| Gomme de Xanthane | 0.2% |
| Acrylate de butyle/acide acrylique/acide méthacrylique | 3.0% |
| Conservateur | 0.4% |
| Eau | 96.4% |

On applique 1g de formule 3 sur 2.5 g de cheveux châtains européens.
On applique 1g de formule témoin (formule 4) sur 2.5 g de cheveux châtains européens de même nature.
La mèche avec la formule 3 est plus fixée.

## Revendications

1. Composition cosmétique capillaire, **caractérisée en ce qu'**elle comprend dans un milieu cosmétiquement acceptable, des particules creuses de matériau organosiliconé et au moins un polymère fixant non siliconé.

2. Composition selon la revendication 1, **caractérisée en ce que** les particules creuses sont des portions de sphères creuses constituées d'un matériau organosiliconé, présentant un diamètre moyen allant de 0,05 µm à 10 µm.

3. Composition selon la revendication 1, **caractérisée en ce que** les particules creuses de matériau organosiliconé sont présentes à des teneurs comprises entre 0,05 et 20% en poids du poids total de la composition.

4. Composition selon l'une quelconque des revendications précédentes, **caractérisée en ce que** le polymère fixant non siliconé est choisi parmi les polymères fixants non siliconés cationiques, anioniques, amphotères, non ioniques et leurs mélanges.

5. Composition selon la revendication 4, **caractérisée en ce que** le polymère fixant non siliconé cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

6. Composition selon la revendication 4, **caractérisée en ce que** le polymère fixant non siliconé anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques et les polyuréthanes anioniques.

7. Composition selon la revendication 4, **caractérisée en ce que** le polymère fixant non siliconé amphotère est choisi parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁-C₅)vinyléther/anhydride maléique modifiés et les polyuréthanes amphotères.

8. Composition selon la revendication 4, **caractérisée en ce que** le polymère fixant non siliconé non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame et les polyuréthanes non ioniques.

9. Composition selon l'une quelconque des revendications précédentes, **caractérisée en ce que** le polymère fixant non siliconé est présent dans la composition à des teneurs comprises entre 0,01 et 20% en poids du poids total de la composition.

10. Composition selon la revendication 9, **caractérisée en ce que** le polymère fixant non siliconé est présent dans la composition à des teneurs comprises entre 0,05 et 15% en poids du poids total de la composition.

11. Composition selon la revendication 9, **caractérisée en ce que** le polymère fixant non siliconé est présent dans la composition à des teneurs comprises entre 0,1 et 10% en poids du poids total de la composition.

12. Composition selon l'une quelconque des revendications précédentes, **caractérisée en ce qu'**elle comprend en outre au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques ou zwitterioniques autres que les polymères fixants non siliconés figurant dans les revendications précédentes, les huiles minérales, végétales ou synthétiques, les agents et tout autre additif classiquement utilisé dans les compositions cosmétiques, les agents anti-pelliculaires, les agents anti-chute, les colorants, les pigments, les agents hydratants, les réducteurs, les oxydants.

13. Procédé cosmétique capillaire, **caractérisé en ce qu'**il comprend l'application sur les cheveux d'une composition selon l'une quelconque des revendications 1 à 12.

14. Utilisation d'une composition selon l'une quelconque des revendications 1 à 12 pour conférer de la brillance aux cheveux.
